(19) Europäisches Patentamt
European Patent Office
Office européen des brevets

(11) Publication number: **0 076 669**
**A1**

(12) **EUROPEAN PATENT APPLICATION**

(21) Application number: **82305244.4**

(22) Date of filing: **01.10.82**

(51) Int. Cl.³: **C 07 C 87/457**
C 07 C 87/60, C 07 C 91/16
C 07 C 91/28, C 07 C 93/14
C 07 C 121/78, C 07 C 149/42
A 61 K 31/135

(30) Priority: **05.10.81 GB 8130009**

(43) Date of publication of application:
**13.04.83 Bulletin 83/15**

(84) Designated Contracting States:
**AT BE CH DE FR GB IT LI LU NL SE**

(71) Applicant: **KEFALAS A/S**
**7-9 Ottiliavej**
**DK-2500 Copenhagen-Valby(DK)**

(72) Inventor: **Bogeso, Klaus, Peter**
**15, Molleaaparken**
**DK-2800 Lyngby(DE)**

(74) Representative: **Pennant, Pyers et al,**
**Stevens, Hewlett & Perkins 5 Quality Court Chancery Lane**
**London, WC2A 1HZ(GB)**

(54) **Novel 3-phenyl-1-indanamines, pharmaceutical compositions and methods of preparation.**

(57) 3-Phenyl-1-indanamines of the formula:

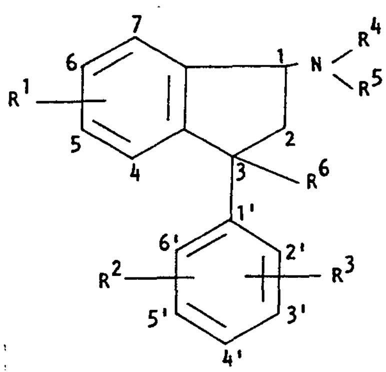

1

wherein $R^1$, $R^2$, $R^3$, $R^4$, $R^5$ and $R^6$ are as defined in Claim 1, and their pharmaceutically acceptable acid addition salts.

The compounds are prepared by methods conventional in the art for preparation of similar compounds.

The compounds have been found to have psychopharmacological activity, especially thymoleptic activity.

Pharmaceutical compositions containing said compounds, and methods for the treatment of psychic disorders using same are described.

EP 0 076 669 A1

0076669

3.9.1982
phn/rr

# Novel 3-Phenyl-1-Indanamines, Pharmaceutical Compositions and Methods of Preparation

The present invention relates to novel 3-phenyl-1-indanamines which have pronounced psychopharmacological activity, especially thymoleptic activity and, at the same time, a low degree of undesired side-effects, methods for the preparation of said indanamines, pharmaceutical compositions containing same, and methods for the treatment of psychic disorders, especially depressions, by administering a therapeutically active amount of one of said indanamines to mammals, including human beings.

The novel 3-phenyl-1-indanamines of the present invention are represented by the following formula:

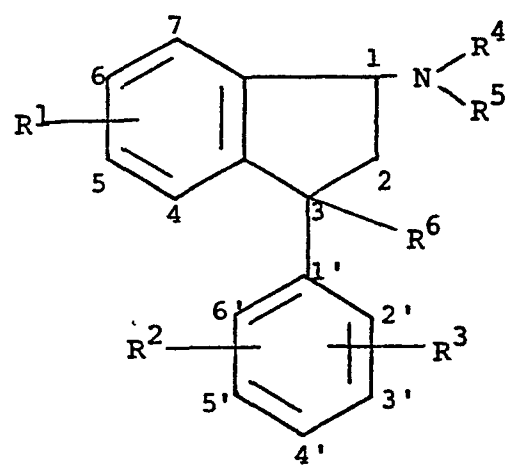

I

wherein $R^1$, $R^2$ and $R^3$ each represents hydrogen, halogen, lower alkyl, lower alkyloxy, hydroxy, lower alkylmercapto, cyano, trifluoromethyl, amino, acylamino, lower alkylamino or nitro, provided that $R^1$, $R^2$ and $R^3$ may not represent hydrogen at the same time, $R^4$ and $R^5$ represent each hydrogen, lower alkyl or lower alkenyl, optionally substituted with hydroxy, optionally substituted cycloalkyl (3-6 C-atoms) or optionally substituted phenyl, $R^6$ represents hydrogen or hydroxy, any hydroxy group present in the indanamine of Formula I being optionally esterified with an aliphatic carboxylic acid having from two to twenty-four carbon atoms inclusive, as well as their pharmaceutically acceptable ancid addition salts.
Some 1-amino-3-phenylindanes have been described as analgetic, spasmolytic and coronary dilating agents (see Adv.Drug Res. 4, 175-180 (1967) ).

Recently, other 1-amino-3-phenylindanes have been described in German Offenlegungsschrift 2 339 715 as having tranquilizing properties.

According to the present invention it has been found that the indanamines of Formula I, as well as their pharmaceutically acceptable acid addition salts, show a thymoleptic profile in test animals, such as rodents, and are very potent inhibitors of catecholamine uptake in vitro. They are active uptake inhibitors of both 5-hydroxytryptamine (5-HT), dopamine (DA) and noradrenaline (NA). All these effects indicate good effectiveness in the treatment of depressions, especially endogenic depressions.
The indanamines of Formula I exist as geometric isomers of the cis-trans- type, and the thymoleptic activity is found in both isomers, but often to a different degree. Each of the geometric isomers exists moreover as optically active isomers, and also these isomers may have the thymoleptic activity to a different degree.

All the aforesaid isomers fall within the scope of the present invention. - Also the methods of isolation of such isomers wellknown to the art fall within the scope of the present invention.

The terms lower alkyl, lower alkenyl, lower alkyloxy and lower alkylmercapto mean such groups - branched or unbranched -having from one to six carbon atoms inclusive, preferably one to three carbon atoms.

Examples of such groups are methyl, ethyl, n-propyl, isopropyl, n-butyl, iso-butyl, n-amyl, n-hexyl, methoxy, ethoxy, propoxy, allyl, 2-butyl, methylmercapto, ethylmercapto, or the like.

When one or more of $R^1$, $R^2$ and $R^3$ represent halogen they are preferably chlorine, bromine or fluorine.

The compounds of Formula I which are most preferable as to pharmacuetical activity fall in two groups. - One group consists of allmost equipotential inhibitors of both DA-, 5-HT-and NA- uptake ; and the other group of much more potent 5-HT uptake inhibitors than DA-or NA- uptake inhibitors.

Preferred examples of compounds of Formula I belonging to the first group are trans-isomers wherein $R^1$ = H or methoxy, $R^2$ and $R^3$ = 3,4-dichloro, $R^4$ = methyl or ethyl, $R^5$ = H or ethyl, and $R^6$ = H.

Preferred examples of compounds belonging to the second group are cis- and trans-isomers of compounds wherein $R^1$ = H, $R^2$ and $R^3$ = 2,4-dichloro, H,4-bromo, H,4-chloro, $R^4$ = H or methyl, $R^5$ = methyl and $R^6$ = H.

This invention also includes pharmaceutically acceptable salts of the compounds of Formula I formed with non-toxic organic acids. Such salts are easily prepared by methods known to the art. -The base is reacted with either the calculated amount of organic or inorganic acid in an aqueous miscible solvent, such as acetone or ethanol, with isolation of the salt by concentration and cooling, or an excess of the acid in aqueous inmiscible solvent, such as ethyl ether or chloroform, with the desired salt separating directly.

Examplary of such organic salts are those with maleic, fumaric, benzoic, ascorbic, pamoic, succinic, oxalic, bis methylene-salicylic, methanesulfonic, ethanedisulfonic, acetic, propionic, tartaric, salicylic, citric, gluconic, lactic, malic, mandelic, cannamic, citraconic, aspartic, stearic, palmitic, itaconic, glycolic, p-amino-benzoic, glutamic, benzene sulfonic and theophylline acetic acids, as well as the 8-halo-theophyllines, for example 8-bromo-theophylline.

Examplary of such inorganic salts are those with hydrochloric, hydrobromic, sulfuric, sulfamic, phosphoric and nitric acids. Of course, these salts may also be prepared by the classical method of double decomposition of appropriate salts, which is wellknown to the art.

The compounds of Formula I as well as the pharmaceutically acceptable acid addition salts thereof may be administered both orally and parenterally, for example in the form of tablets, capsules, powders, syrups or solutions for injection.

The invention moreover relates to a method for the preparation of the novel indanamines of Formula I, which comprises

a) reacting a compound of the following formula:

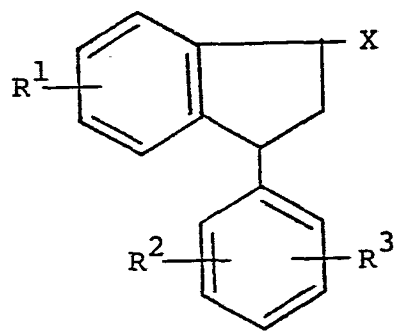

II

wherein $R^1$, $R^2$ and $R^3$ are as defined above, and X is halogen, preferably chlorine,or $-OSO_2R$, wherein R is $CH_3$ or p-tolyl, with an amine of formula $HNR^4R^5$, wherein $R^4$ and $R^5$ are as defined above

or

b) reducing a Schiff-base of the following formula :

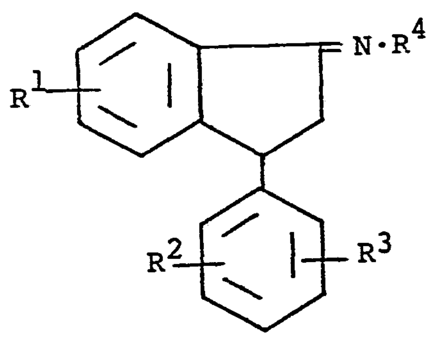

III

with a reducing agent such as $NaBH_4$ or hydrogen in the presence of a metal catalyst, such as palladium on charcoal or $PtO_2$,

or

c) alkylating a compound of the following formula:

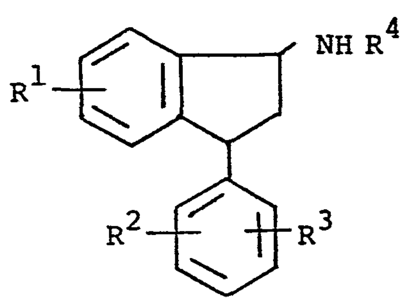

IV

wherein $R^1$, $R^2$, $R^3$ and $R^4$ are as defined above, with either a compound $R^5 \cdot X$ wherein $R^5$ and X are as defined above, or by reductive alkylation with an aldehyde $R^{5'} \cdot CHO$ or ketone $R^{6'}CO \cdot R^{7'}$ or a carboxylic acid -$NaBH_4$ complex, wherein $R^{5'}$ represents optionally substituted alkyl or alkenyl having one carbon atom less than $R^5$, and $R^6$ and $R^7$ together with the carbon atom from the keto group form a branched optionally substituted lower alkyl- or alkenyl group,

or

d) reducing a compound of the following formula:

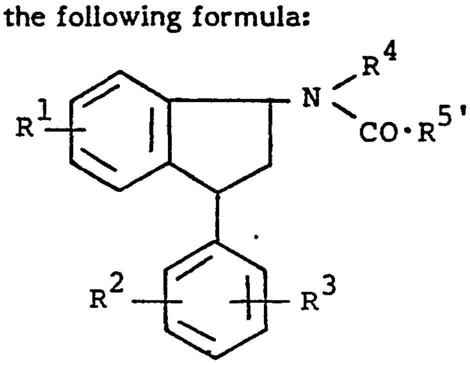

V

wherein $R^1$, $R^2$, $R^3$,$R^4$ and $R^5$ are as defined above, or with a reducing agent such as $LiAlH_4$,

or

e) reacting an indanone of the following formula:

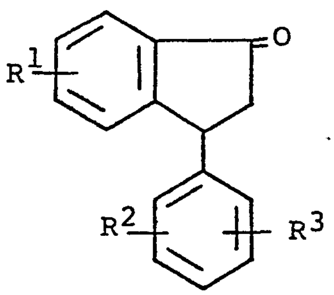

VI

wherein $R^1$, $R^2$ and $R^3$ are as defined above, with an amine of the formula $R^4 \cdot NH$ $R^5$, wherein $R^4$ and $R^5$ are as defined above, in the presence of hydrogen and a metal catalyst,

or

f) reacting a compound of the following formula:

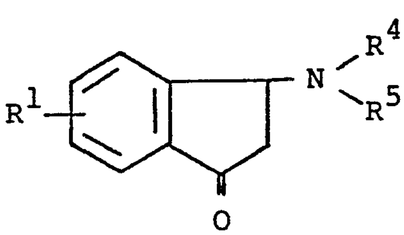

VII

wherein $R^1$, $R^4$ and $R^5$ are as defined above, with a Grignard- or lithium reagent of the formula 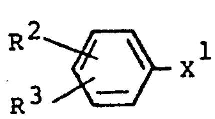

wherein $R^2$ and $R^3$ are as defined above, and $X^1$ is Mghal or Li, followed by hydrolysis of the complex formed,

or

g) reducing a compound of the following formula:

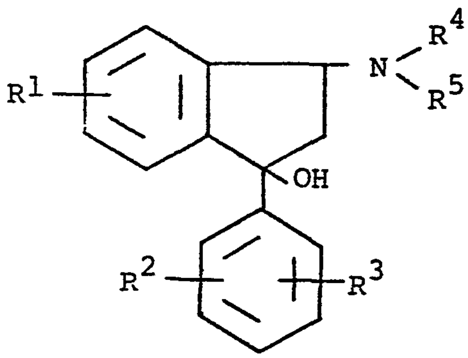

VIII

wherein $R^1$, $R^2$, $R^3$, $R^4$ and $R^5$ are as defined above,

or

h) reducing an indene of the following formula:

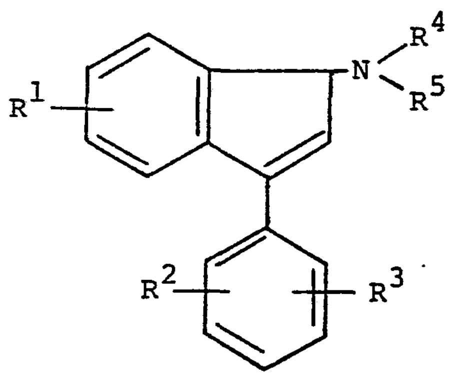

IX

wherein $R^1$, $R^2$, $R^3$, $R^4$ and $R^5$ are as defined above, or

i) cleaving a methoxy group in a compound of Formula I, wherein one of $R^1$, $R^2$ or $R^3$ is methoxy, to give the corresponding hydroxy substituted compound,

whereupon the indanamine of Formula I formed by the reaction is isolated in the form of the free base or in the form of a pharmaceutically acceptable acid addition salt and, if desired, separated in the individual isomers in conventional manner, any hydroxy group present optionally being esterified with a reactive derivative of an aliphatic carboxylic acid having from one to twenty-four carbon atoms inclusive.

The methods of preparation according to the invention are summarized in the following Scheme I and Scheme II.

## SCHEME 1

Methods of Preparation

Scheme I ($R^6$ = H)

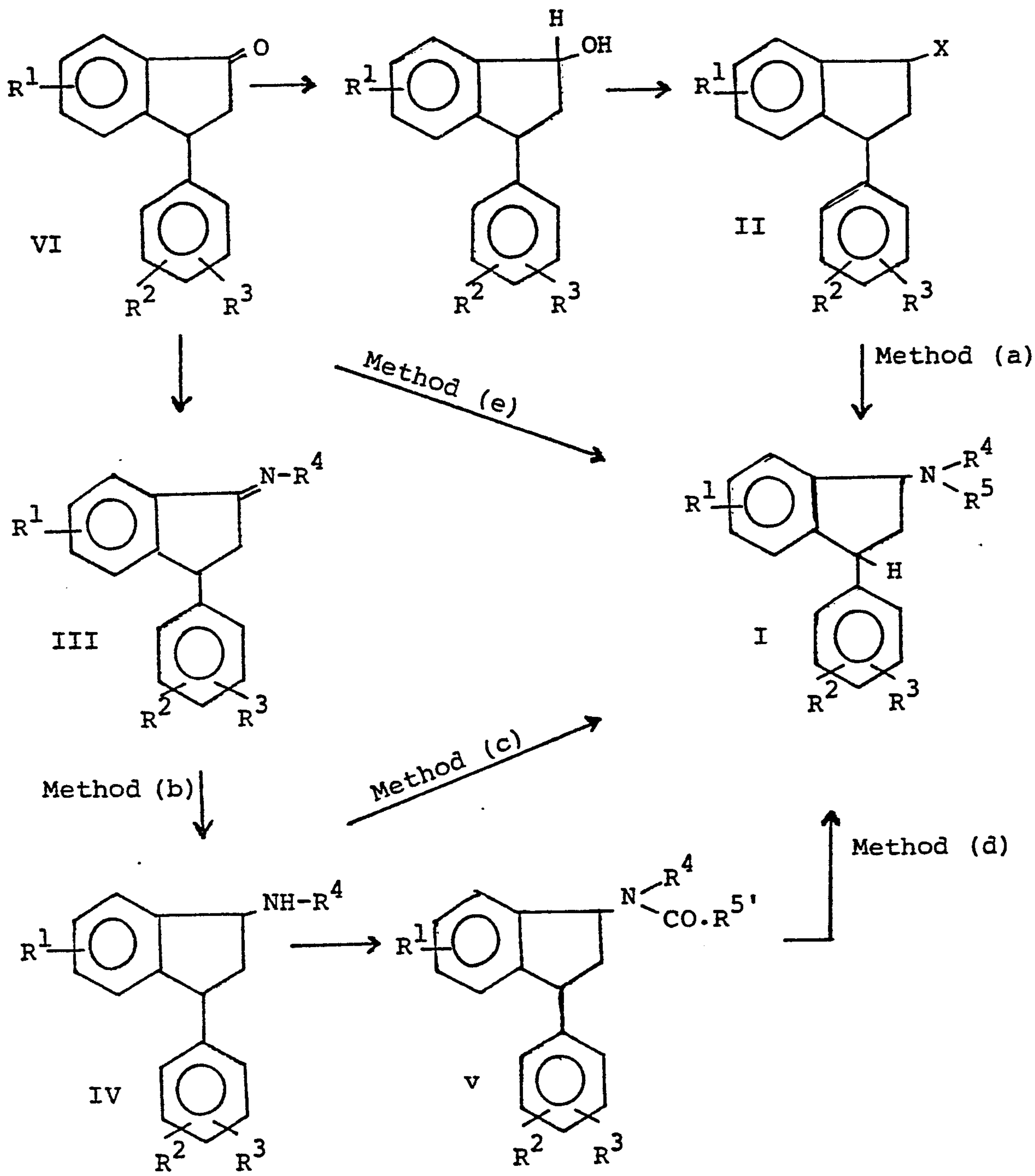

SCHEME II

(R$^6$ = OH or H)

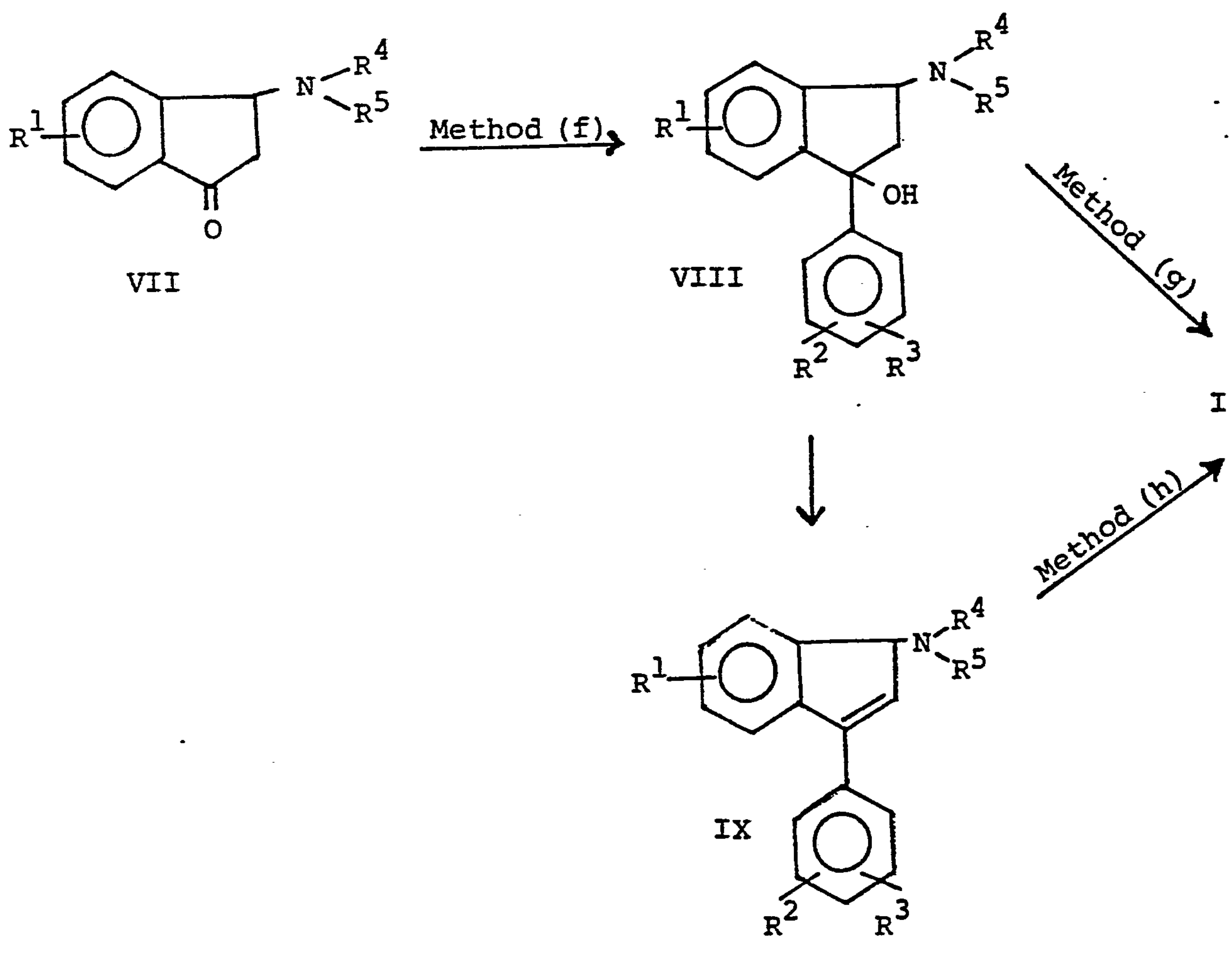


VII VIII IX I

Method a) is preferably carried out by reacting the compound of Formula II with the appropriate amine in a suitable inert organic solvent such as a lower alkanol, e.g. ethanol, at elevated temperatures in a steel autoclave. When the amine itself has a sufficiently high boiling point the reaction can be carried out at atmospheric pressure at reflux temperature in the presence of an inert solvent such as an alcohol or toluene. When X is halogen the indanamine of Formula I is mostly a mixture of isomers containing a larger amount of the trans-isomer. When X is $-OSO_2R$, an isomeric mixture with a considerably higher cis-content is obtained.

In method b) the reduction of the compound of Formula III is preferably carried out in an inert solvent such as an alkanol, e.g. methanol or ethanol, at room temperature, using $NaBH_4$ as a reducing agent. This method gives almost exclusively the cis-isomers.

In method c) the reaction with the compound $R^5$ ·X is preferably carried out in an inert solvent such as acetone, ether, ethanol or the like, in the presence of base (excess amine or an alkali metal carbonate) at reflux temperature.
The reductive alkylations according to method c) are preferably carried out by reacting the amine IV with an aldehyde or ketone in an alkanol at pH 6-8 at room temperature, using NaCN $BH_3$ or $NaBH_4$ as reducing agents. Reductive alkylations with carboxylic acids and $NaB_4$ can according to the invention be carried out as described by Marchine et al., J.Org.Chem. 1975, 40, 3453. Methylations are preferably carried out by the Leuckart-Wallach reduction using formaldehyde-formic acid mixture.

In method d) is the reduction of the amide of Formula V preferably carried out in an inert solvent such as diethylether, with a suitable reducing agent, e.g. $LiAlH_4$.

The reductive amination of the indanones of Formula IV is according to method e) preferably carried out in an inert solvent with hydrogen under pressure at elevated temperatures using a suitable catalyst such as Raney nickel, palladium on charcoal, or the like. This method leads almost exclusively to cis-isomers.

In method f) is the reaction of the compound of Formula VII with the organo-metallic reagent preferably carried out in an inert solvent such as diethylether or tetrahydrofuran at reflux temperature. The following hydrolysis is carried out in conventional manner.

In method g) is the reduction of the indanol VIII preferably carried out in a solvent such as acetic acid using a suitable reducing agent such as a mixture of hydroiodic acid and red phosphorus.

In method h) is the reduction of the indene IX preferably carried out in an inert solvent such as an alkanol in the presence of hydrogen at low pressure using a suitable metal catalyst such as palladium on charcoal. This method leads almost exclusively to cis-isomers.

In method i) is the cleaving of the methoxy group preferably carried out in 48% hydrobromic acid at reflux temperature or with boron tribromide in methylene chloride at room temperature or lower temperature.

All the novel compounds of Formula I according to the invention can be made by method a); the resulting mixture of isomers may then be separated in the individual isomers by fractional crystallisation from a suitable solvent or solvent mixture.
If larger quantities of cis-isomers are wanted, methods b) and g) are to be preferred.

The various optionally substituted indanones of Formula VI and the indanols prepared therefrom (see Scheme I), and the intermediates of Formula II were prepared according to methods described in our copending European Patent Application, Serial No. 81300785.3.

The following indanones and indanols in Table I are new substances.

Table 1

| $R^1$ | $R^2$ | $R^3$ | Indanones MP | Indanols MP |
|---|---|---|---|---|
| H | 3'-Cl | H | 110°C | 100°C |
| H | 4'-Cl | H | 74-78°C (not new) | 92-94°C |
| H | 3'-F | 4'-F | 104-106°C | oil, not purified |
| H | 3'-Cl | 4'-F | 166-169°C | 70-75°C |
| 6-F | 3'-Cl | 4'-Cl | 118-120°C | not purified |
| 6-$CH_3O$ | 3'-Cl | 4'-Cl | oil, not purified | oil, not purified |

The Schiff-bases of Formula III are conveniently prepared by reacting the indanones IV with a primary amine in dry toluene at $0^{o}C$ in the presence of titantetrachloride.

The amides of Formula V were prepared by reacting a secondary amine of Formula IV with an acid chloride in the presence of a base.

The intermediates of Formula VII were prepared by reacting the corresponding 3-bromo-indanones with an amine in a suitable solvent, such as ethanol, at lower temperatures.

The indenes of Formula IX were prepared by dehydrating the indanols of Formula VIII with a suitable dehydrating agent, such as hydrochloric acid, acetic acid or phosphorus oxychloride in pyridine.

The optional esterification of any hydroxy group or groups present in the indanamines of Formula I may, according to the invention, conveniently be carried out with a reactive derivative of the alophatic carboxylic acid having from one to twenty-four carbon atoms inclusive, such as an acid halide, especially acid chloride, or anhydride, including mixed anhydrides. As especially suitable acids may be mentioned acetic acid, propionic acid, decanoic acid, palmitic acid and behenic acid, or the like.

The methods of preparing the indanamines of Formula I are in the following illustrated by specific examples which, however, must not be construed as limiting for the invention.

EXAMPLE 1

Method a)

Cis- and trans-N-methyl-3-(3',4'-dichlorophenyl)-1-indanamine,

The starting material, 1-chloro-3-(3',4'-dichlorophenyl)-indane, was made in the following way:

A solution of 400 grams of 3-(3',4'-dichlorophenyl)-1-indanol in 1 liter of dry toluene was cooled in ice-water, and 200 milliliters of thionyl chloride were added, keeping the temperature below 30°. The reaction mixture was stirred at room temperature for 15 minutes and was thereupon heated to 60°C and kept at this temperature for 1 hour. The reaction mixture was treated with ice-water, extracted with aqueous sodium carbonate solution and dried over anhydrous magnesium sulfate. After filtration and evaporation in vacuo 400 grams of 1-chloro-3-(3',4'-dichlorophenyl)-indane were obtained and then used in the next step without further purification.

A mixture of 100 grams of 1-chloro-3-(3',4'-dichlorophenyl)-indane and 200 ml 33% methylamine in ethanol was heated overnight at 100°C in a steel autoclave. The reaction mixture was evaporated in vacuo, and the resulting oil was taken up in ether. The ether extract was washed twice with water and then extracted with dilute hydrochloric acid. The acid extract was made alkaline with 10 N sodium hydroxide and then extracted with ether. The ether extract was washed with water, dried over anhydrous potassium carbonate and evaporated in vacuo. Yield: 80 grams of crude product as an oil.

The oil was taken up in 500 ml of ethanol, and the solution was made acid with a saturated solution of oxalic acid in acetone. The resulting oxalate was filtered and recrystallized twice from 2-methoxyethanol. The oxalate was converted to the hydrochloride in conventional manner. The hydrochloride was recrystallized from acetone to give 6 grams of cis-N-methyl-3-(3',4'-dichlorophenyl)-1-indanamine, melting at 230-232°C. (Lu 19-006-c).

CHN calc.: 58.46% ; 4.92% ; 4.26%.
CHN found: 58.33% ; 4.98% ; 4.24%.

The combined mother liquors from the above mentioned oxalate were concentrated in vacuo. The concentrate was converted to the base, and the trans-isomer was isolated as the hydrochloride. After recrystallization from ethylacetate-ethanol-ether there was obtained 20 grams of trans-N-methyl-3-(3',4'-dichlorophenyl)-1-indanamine, melting at 177-179$^{o}$C. (Lu 19-005-c).

CHN calc.: 58.46% ; 4.92% ; 4.26%.
CHN found: 58.12% ; 4.97% ; 4.17%.

EXAMPLE 2
Method b)

Cis N-methyl-3-(4'-fluorophenyl)-1-indanamine.
The starting material, N-methyl-3-(4'-fluorophenyl)-1-indanimine, was prepared in the following way:

A solution of 23 grams of 3-(4'-fluorophenyl)-1-indanone and 30 ml of methylamine in 400 ml of dry toluene was cooled to 0$^{o}$C in a dry ice-acetone bath, and a solution of 6 ml of titantetrachloride in 100 ml of toluene was added dropwise at 0$^{o}$C. The reaction mixture was stirred for 1 hour at 0$^{o}$C and overnight at room temperature. The reaction mixture was filtered and evaporated in vacuum to give 23 grams of N-methyl-3-(4'-fluorophenyl)-1-indanimine which were used in the next step without further purification.
To a solution of 23 grams of crude N-methyl-3-(4'-fluorophenyl)-1-indanimine in 300 ml of methanol were added in small portions 12 grams of sodium boro hydride under cooling in an ice-bath. The reaction mixture was stirred for one hour at room temperature and then evaporated in vacuum. The resulting oil was treated with 200 ml of 2N NaOH and then extracted with ether.
The ether phase was washed with water and extracted with 2N methane sulfonic acid. The acid extract was made alkaline with 10 N NaOH and extracted with ether. The ether extract was washed with brine, dried over anhydrous potassium carbonate and evaporated in vacuum. The resulting oil was taken up in acetone and made acid with a saturated solution of hydrogen chloride in ether.
The crystalline hydrochloride was filtered and recrystallized from methanol acetone to yield 15 grams of pure cis N-methyl-3-(4'-fluorophenyl)-1-indanamine, hydrochloride. MP: 257-258$^{o}$C. (Lu 19-108-c).

CHN calc.: 69.18% ; 6.18% ; 5.04%.
CHN found: 69.15% ; 6.18% ; 4.98%.

EXAMPLE 3

Method c)

Trans-N,N-dimethyl-3-(3-chlorophenyl)-1-indanamine.

The starting material , trans N-methyl-3-(3-chlorophenyl)-1-indanamine, was prepared according to Example 1.

A mixture of 10 grams of N-methyl-3-(3-chlorophenyl)-1-indanamine hydrochloride, 7 ml of 34% formaldehyde and 30 ml of formic acid was refluxed for 3 hours.

The reaction mixture was poured on ice and made strongly alkaline with 10 N sodium hydroxide.The base was extracted with ether and purified by extraction with dilute hydrochloric acid, liberation of the base with sodium hydroxide solution and re-extraction with ether. The ether phase was dried and evaporated in vacuum. The resulting oil was taken up in acetone and made acid with a saturated solution of hydrogen chloride in ether. The resulting hydrochloride was recrystallized from acetone-methanol (1:1) to give 6.5 grams of trans N,N-dimethyl-3-(3-chlorophenyl)-1-indanamine hydrochloride, melting at 201-202$^{o}$C.

CHN calc.: 66.23% ; 6.23% ; 4.55%.

CHN found: 65.82% ; 6.20% ; 4.53%.

EXAMPLE 4

(Method d)

Trans-N-ethyl-N-methyl-3-(3'-chloro-4'-fluorophenyl)-1-indanamine.

The starting material, N-acetyl-N-methyl-3-(3'-chloro-4'-fluorophenyl)-1-indanamine, was made in the following way:

7 grams of trans-N-methyl-3-(3'-chloro-4'-fluorophenyl)-1-indanamine hydrochloride (Lu 18-085-c, made by method a) were added to a separatory funnel containing 200 ml of ether and a solution of 10 grams of potassium carbonate in 200 ml of water. The mixture was shaken until two clear phases were obtained, whereupon 5 ml of acetyl chloride were added. The mixture was shaken until the $CO_2$-evolution ended. The phases were separated and the organic phase was extracted with 1 N hydrochloric acid, washed and dried over anhydrous magnesium sulfate. After filtration and evaporation in vacuum 7.5 grams of crude trans N-acetyl-N-methyl-3-(3'-chloro-4'-fluorophenyl)-1-indanamine were obtained and used without further purification in the next step.

To 7.5 grams of crude N-acetyl-N-methyl-3-(3'-chloro-4'-fluorophenyl)-1-indanamine in 200 ml of ether was added 1 gram of lithium aluminium hydride. The mixture was refluxed for two hours and then hydrolyzed with water. After filtration the base was extracted from the ether phase with dilute hydrochloric acid, whereupon the base was liberated with 10 N NaOH, extracted with ether, dried over anhydrous potassium carbonate, filtered and evaporated in vacuum. The resulting oil was taken up in ethyl acetate and made acid with a saturated solution of oxalic acid in acetone. The oxalate was filtered and dried to yield 3 grams of pure trans N-ethyl-N-methyl-N-3-(3'-chloro-4'-fluorophenyl)-indanamine as the oxalate.

Melting Point: 97-99°C. (Lu 19-105-o).

CHN calc.; 60.98% ; 5.39% ; 3.56%.

CHN found: 60.54% ; 5.44% ; 3.42%.

## EXAMPLE 5
## (Method f)

### N,N-Dimethyl-3-(3-chloro-4-fluorophenyl)-3-hydroxy-1-indanamine.

The starting material, N,N-dimethyl-3-oxo-1-indanamine, was prepared in the following way:

A solution of 600 ml of 40% dimethylamine in ethanol was cooled in an ice-salt mixture, and 270 grams of 3-bromo-1-indanone in 500 ml of ethyl acetate were added at 5-10°C. The reaction mixture was stirred at room temperature for 15 minutes and then evaporated in vacuum. The resulting oil was taken up in ether, and the base was purified by extraction with dilute hydrochloric acid; liberation of the base with 10 N sodium hydroxide and extraction with methylene chloride. The organic phase was dried over anhydrous $K_2CO_3$ and evaporated in vacuum to give 230 grams of an oil. The oil was taken up in petroleum ether, from which 196 grams of N,N-dimethyl-3-oxo-1-indanamine were obtained (melting at 50-52°C).

A solution of 3-chloro-4-fluorophenyl magnesium bromide in ether was made from 63 grams of 3-chloro-4-fluoro-bromobenzene and 7.4 grams of magnesium turnings in 160 ml of ether. A solution of 44 grams of N,N-dimethyl-3-oxo-1-indanamine was added, and the reaction mixture was refluxed for two hours. The reaction mixture was hydrolyzed with ice-water and ammonium chloride and extracted with ether. The organic phase was washed with water, dried over anhydrous $MgSO_4$ and evaporated in vacuum. The oil was taken up in ether and the base was purified by extraction with dilute acetic acid; liberation of the base with 10 N sodium hydroxyde, and re-extraction with ether. The organic phase was dried over anhydrous $K_2CO_3$ and evaporated in vacuum to give 66 grams of an oil. The oil was taken up in hexane and

cooled to give 53 grams of crude N,N-dimethyl-3-(3-chloro-4-fluorophenyl)-3-hydroxy-1-indanamine, melting at 94-96°C.

13 grams were purified by conversion to the oxalate salt in acetone, and recrystallization of this from ethanol-ether (1:1) to give 10.5 grams of pure oxalate. This was converted to the base, which was crystallized from petroleum ether to give 7.3 grams of pure N,N-dimethyl-3-(3-chloro-4-fluorophenyl)-3-hydroxy-1-indanamine, melting at 98-100°C. (Lu 19-114).

CHN calc.: 66.77% ; 5.62% ; 4.58%.

CHN found: 66.38% ; 5.59% ; 4.49%.

In similar way was prepared:

N,N-Dimethyl-3-(3,4-dichlorophenyl)-3-hydroxy-1-indanamine,

melting at 117-119°C. (Lu 19-034).

EXAMPLE 6

(Method g)

Cis and trans N,N-dimethyl-3-(3-chloro-4-fluorophenyl)-1-indanamine.

A mixture of 5 grams of N,N-dimethyl-3-(3-chloro-4-fluorophenyl)-3-hydroxy-1-indanamine, 40 ml of acetic acid, 30 ml of 57% hydroiodic acid, 3 grams of red phosphorous and 1.5 ml of water was refluxed for 4 hours.

The reaction mixture was filtered, poured on ice and made basic with concentrated aqueous ammonia. The base was extracted with ether and purified by extraction with dilute acetic acid; liberation of the base with 10 N NaOH -solution and extraction with ether. The ether extract was dried with anhydrous potassium carbonate and evaporated in vacuum to give 4 grams of crude base. This was taken up in ethyl acetate, and a saturated solution of hydrogen chloride in ether was added. The hydrochloride crystallized overnight in refrigerator to give 3.7 grams of a mixture of the hydrochlorides of cis and trans N,N-dimethyl-3-(3-chloro-4-fluorophenyl)-1-indanamine. (Lu 19-100-c and Lu 19-101-c).

The isomeric mixture has a proportion between cis:trans isomers of 2:1 (TLC using pure Lu 19-100-c and Lu 19-101-c prepared by method a as standards).

EXAMPLE 7

(Method h)

Cis- N,N-dimethyl-3-(3-chloro-4-fluorophenyl)-1-indanamine.

The starting material, N,N-dimethyl-3-(3-chloro-4-fluorophenyl)-1-indene amine, was prepared in the following way:

A solution of 7 grams of N,N-dimethyl-3-(3-chloro-4-fluorophenyl)-3-hydroxy-1-indanamine in 40 ml of concentrated hydrochloric acid and 20 ml of acetic acid was refluxed for two hours. The reaction mixture was evaporated to dryness in vacuum and treated with ethyl acetate to give 4.5 grams of N,N-dimethyl-3-(3-chloro-4-fluorophenyl)-1-indenamine,HCL melting at 138-140°C.
To 2.1 grams of N,N-dimethyl-3-(3-chloro-4-fluorophenyl)-1-indenamine hydrochloride in 60 ml of ethanol was added 1 gram of 5% Pd/C, and the mixture was hydrogenated in a Parr-apparatus at 3 atm. for 1.5 hours. After filtration and evaporation in vacuum the hydrochloride was treated with ethyl acetate to give 1.4 grams of pure cis- N,N-dimethyl-3-(3-chloro-4-fluorophenyl)-1-indanamine, hydrochloride (Lu 19-101-c), melting at 189-191°C.

EXAMPLE 8

Cis- N-methyl-3-(3,4-difluorophenyl)-1-indanamine.

A mixture of 5 grams of 3-(3,4-difluorophenyl)-1-indanone, 25 ml of methanol, 25 grams of methylamine and 1 gram of Raney-Nickel was hydrogenated at 30 atm. and 50°C for 3 hours. After filtration and evaporation the resulting oil was taken up in ethyl acetate and made acid with a saturated solution of hydrogen chloride in ether. The resulting hydrochloride was recrystallized from acetone to give 3 grams of cis-N-methyl-3-(3,4-difluorophenyl)-1-indanamine, (Lu 19-104-c), melting at 240-243°C.
CHN calc.: 64.97% ; 5.46% ; 4.74%.
CHN found: 64.84% ; 5.46% ; 4.68%.

EXAMPLE 9
(Method i)

Trans- N,N-dimethyl-3-(3,4-dichlorophenyl)-6-hydroxy-1-indanamine, hydrobromide.

A mixture of 5.9 grams of trans N,N-dimethyl-3-(3,4-dichlorophenyl)-6-methoxy-1-indanamine, hydrochloride (prepared by Method a, see Example 1) and 100 ml of 48% hydrobromic acid was refluxed for 3.5 h. The solution was evaporated in vacuo, and the residue was treated with ethanol and ether and crystallized to give 4.2 grams of the title compound. After two recrystallizations from methanol-ether, 3.2 grams of trans N,N-dimethyl-3-(3,4-dichlorophenyl)-6-hydroxy-1-indanamine, hydrobromide were obtained. MP: 217-221°C (Lu 20-031-B).
CHN calc.: 50.64%; 4.51%; 3.48%.
CHN found: 50.34%; 4.41%; 3.39%.

EXAMPLE 10

Optical resolution of enantiomers. (+) and (-) trans N-methyl-3-(3,4-dichlorophenyl)-1--indanamine.

To a solution of 18.8 grams of (±) trans N-methyl-3-(3,4-dichlorophenyl)-1-indanamine (Lu 19-005), prepared as described in Example 1, in 30 ml of ethyl acetate was added a solution of 6 grams of (+)-0,0'-dibenzoyl-D-tartaric acid hydrate in 20 ml of ethyl acetate. After crystallization at room temperature there was obtained 16 grams of the (+) -0,0'-dibenzoyl-D-tartaric acid salt. MP: 169-170°C. After recrystallization from methanol-ethyl acetate there was obtained 13 grams. MP: 169-170°C. The salt was converted to the base. $[\alpha]_D^{22}$ = +24.48° (C=4.09, $CH_3OH$). The base was dissolved in acetone and converted to the HCl salt by addition of a saturated solution of HCl in ether. The hydrochloride was recrystallized from methanol-acetone to give 5.5 grams of (+) trans N-methyl-3-(3,4-dichlorophenyl)-1-indanamine, hydrochloride. MP: 184-185°C. (Lu 20-042-c).

CHN calc.: 58.46%; 4.92%; 4.26%.

CHN found: 58.87%; 4.88%; 4.25%.

The first filtrate from the isolation of the (+) - 0,0'-dibenzoyl-D-tartaric acid salt was converted to the base. The base (8.7 grams) was dissolved in 20 ml of ethyl acetate, whereupon 5.6 grams of (-) - 0,0'-dibenzoyl-L-tartaric acid hydrate were added. After crystallization there was obtained 7 grams of the (-) -0,0'-dibenzoyl-L-tartaric acid salt. MP: 173-174°C. The salt was converted to the base. $[\alpha]_D^{22}$ = -23.36° (C= 3.85, $CH_3OH$).

The base was dissolved in acetone and converted to the HCl salt by addition of a saturated solution of HCl in ether to give 3 grams of (-) trans N-methyl-3-(3,4-dichlorophenyl)-1-indanamine, hydrochloride. MP: 182-183°C. (Lu 20-043-c).

CHN calc.: 58.46%; 4.92%; 4.26%.

CHN found: 58.83%; 4.94%; 4.27%.

The compounds of Formula I made according to the invention appear from the following Table 2.

$R^6$ = H : $R^6$ = OH see Example 5

x) > 95% pure isomers are only stated as "cis" or "trans"

| Code No. | $R^1$ | $R^2$ | $R^3$ | $R^4$ | $R^5$ | Isomerism x) | Salt/Base | M.P. °C |
|---|---|---|---|---|---|---|---|---|
| 19-005-c | H | 3'-Cl | 4'-Cl | H | $CH_3$ | (90%)trans | Hydrochloride | 177-179 |
| 19-006-c | H | 3'-Cl | 4'-Cl | H | $CH_3$ | cis | Hydrochloride | 230-232 |
| 18-118 | H | 3'-Cl | 4'-Cl | $CH_3$ | $CH_3$ | trans | Base | 78-79 |
| 18-122-c | H | 3'-Cl | 4'-Cl | $CH_3$ | $CH_3$ | cis | Hydrochloride, hydrate | 180-184 |
| 19-040-c | H | 3'-Cl | 4'-Cl | H | $C_2H_5$ | trans | Hydrochloride | 227-229 |
| 19-041-c | H | 3'-Cl | 4'-Cl | H | $C_2H_5$ | cis | Hydrochloride | 268-270 |
| 18-187 | H | 3'-Cl | 4'-Cl | $C_2H_5$ | $C_2H_5$ | trans(94%) | Hydrochloride | 157-158 |
| 18-188-c | H | 3'-Cl | 4'-Cl | $C_2H_5$ | $C_2H_5$ | cis | Hydrochloride | 226-227 |
| 19-042-c | H | 3'-Cl | 4'-Cl | $C_3H_7$ | $C_3H_7$ | trans | Hydrochloride | 219-222 |
| 19-043-c | H | 3'-Cl | 4'-Cl | $C_3H_7$ | $C_3H_7$ | cis | Hydrochloride | 190-193 |
| 19-030-o | H | 3'-Cl | 4'-Cl | H | H | isomeric mix. ~60:40 | Oxalate | 163-166 |
| 19-073-c | H | 3'-Cl | 4'-Cl | H | $-CH_2CH_2OH$ | cis | Hydrochloride | 235-238 |
| 19-074-c | H | 3'-Cl | 4'-Cl | H | $CH_2CH_2OH$ | trans | Hydrochloride | 167-169 |
| 19-082-c | H | 3'-Cl | 4'-Cl | $CH_2CH_2OH$ | $CH_2CH_2OH$ | trans | Hydrochloride | 164-166 |
| 19-071-c | H | 3'-Cl | 4'-Cl | H | $-CH_2C_6H_5$ | trans | Hydrochloride | 269-272 |
| 19-072-c | H | 3'-Cl | 4'-Cl | H | $-CH_2C_6H_5$ | cis | Hydrochloride | 230-233 |

Table 2 (cont'd)

| Code No. | $R^1$ | $R^2$ | $R^3$ | $R^4$ | $R^5$ | Isomerism x) | Salt/Base | M.P. °C |
|---|---|---|---|---|---|---|---|---|
| 19-078-c | H | 3'-Cl | 4'-Cl | H | $-CH_2CH_2C_6H_5$ | trans (90%) | Hydrochloride | 241-243 |
| 19-079-o | H | 3'-Cl | 4'-Cl | H | $-CH_2CH_2C_6H_5$ | cis (90%) | Oxalate | 228-230 |
| 19-083-o | H | 3'-Cl | 4'-Cl | H | $CH_2CH{=}CH{\cdot}C_6H_5$ | trans (90%) | Oxalate | 220-222 |
| 19-097 | 6-F | 4'-F | H | H | $CH_3$ | trans | Base | 99-101 |
| 13-164 | 6-F | 4'-F | H | H | $CH_3$ | cis | Base | 34-36 |
| 13-114-c | 6-F | 4'-F | H | $CH_3$ | $CH_3$ | trans | Hydrochloride | 238-242 |
| 18-147 | 6-F | 4'-F | H | $CH_3$ | $CH_3$ | cis | Base | 77-80 |
| 18-149-c | 6-F | 4'-F | H | $C_2H_5$ | $C_2H_5$ | trans | Hydrochloride | 186-188 |
| 18-148-c | 6-F | 4'-F | H | $C_2H_5$ | $C_2H_5$ | cis | Hydrochloride | 230-233 |
| 18-143-c | 6-F | 4'-F | H | $C_3H_7$ | $C_3H_7$ | trans | Hydrochloride | 230-231 |
| 18-144-c | 6-F | 4'-F | H | $C_3H_7$ | $C_3H_7$ | cis | Hydrochloride | 221-222 |
| 19-109-c | H | 4'-F | H | H | $CH_3$ | trans | Hydrochloride | 162-163 |
| 19-108-c | H | 4'-F | H | H | $CH_3$ | cis | Hydrochloride | 257-258 |

Table 2 (cont'd)

| Code No. | $R^1$ | $R^2$ | $R^3$ | $R^4$ | $R^5$ | Isomerism x) | Salt/Base | M.P. °C |
|---|---|---|---|---|---|---|---|---|
| 19-007-c | H | 4'-F | H | $CH_3$ | $CH_3$ | trans | Hydrochloride, hydrate | 156-158 |
| 19-008-c | H | 4'-F | H | $CH_3$ | $CH_3$ | cis | Hydrochloride | 207-209 |
| 19-111-c | H | 3'-Cl | H | H | $CH_3$ | trans | Hydrochloride | 159-160 |
| 19-110-c | H | 3'-Cl | H | H | $CH_3$ | cis | Hydrochloride | 215-216 |
| 19-115-c | H | 3'-Cl | H | $CH_3$ | $CH_3$ | trans | Hydrochloride | 201-202 |
| 19-116-c | H | 3'-Cl | H | $CH_3$ | $CH_3$ | cis | Hydrochloride | 198-200 |
| 19-119 | H | 4'-Cl | H | H | $CH_3$ | trans | Base | 67-69 |
| 19-120-c | H | 4'-Cl | H | H | $CH_3$ | cis | Hydrochloride | 246-247 |
| 19-121 | H | 4'-Cl | H | $CH_3$ | $CH_3$ | trans | Base | 79-81 |
| 19-124 | H | 4'-Cl | H | $CH_3$ | $CH_3$ | cis | Base | 78-80 |
| 19-102-c | H | 4'-Br | H | H | $CH_3$ | trans | Hydrochloride | 213-215 |
| 19-103-c | H | 4'-Br | H | H | $CH_3$ | cis | Hydrochloride | 240-242 |
| 19-056 | H | 4'-Br | H | $CH_3$ | $CH_3$ | trans | Base | 75-77 |
| 19-057 | H | 4'-Br | H | $CH_3$ | $CH_3$ | cis | Base | 86-89 |

| Code No. | $R^1$ | $R^2$ | $R^3$ | $R^4$ | $R^5$ | Isomerism x) | Salt/Base | M.P.°C |
|---|---|---|---|---|---|---|---|---|
| 19-098-c | H | 3'-F | 4'-F | H | $CH_3$ | trans (85%) | Hydrochloride | 170-174 |
| 19-104-c | H | 3'-F | 4'-F | H | $CH_3$ | cis | Hydrochloride | 240-243 |
| 19-095-c | H | 3'-F | 4'-F | $CH_3$ | $CH_3$ | trans | Hydrochloride | 180-182 |
| 19-096-o | H | 3'-F | 4'-F | $CH_3$ | $CH_3$ | cis | Oxalate | 165-167 |
| 19-085-c | H | 3'-Cl | 4'-F | H | $CH_3$ | trans | Hydrochloride | 184-185 |
| 19-086-c | H | 3'-Cl | 4'-F | H | $CH_3$ | cis | Hydrochloride | 225-226 |
| 19-100-c | H | 3'-Cl | 4'-F | $CH_3$ | $CH_3$ | trans | Hydrochloride | 215-216 |
| 19-101-c | H | 3'-Cl | 4'-F | $CH_3$ | $CH_3$ | cis | Hydrochloride | 189-191 |
| 19-105-o | H | 3'-Cl | 4'-F | $CH_3$ | $C_2H_5$ | trans | Oxalate | 97-99 |
| 19-099-c | H | 2'-Cl | 4'-Cl | H | $CH_3$ | trans | Hydrochloride | 195-197 |
| 19-113-c | H | 2'-Cl | 4'-Cl | H | $CH_3$ | cis | Hydrochloride | 247-249 |
| 18-136-c | H | 2'-Cl | 4'-Cl | $CH_3$ | $CH_3$ | trans | Hydrochloride | 262-264 |
| 18-137-c | H | 2'-Cl | 4'-Cl | $CH_3$ | $CH_3$ | cis | Hydrochloride | 237-239 |
| 19-092-c | 6-$CH_3$ | 4'-F | H | H | $CH_3$ | trans | Hydrochloride | 222-224 |
| 19-091-c | 6-$CH_3$ | 4'-F | H | H | $CH_3$ | cis | Hydrochloride | 247-250 |

Table 2 (cont'd)

| Code No. | $R^1$ | $R^2$ | $R^3$ | $R^4$ | $R^5$ | Isomerism x) | Salt/Base | M.P. °C |
|---|---|---|---|---|---|---|---|---|
| 18-125-c | 6-$CH_3$ | 4'-F | H | $CH_3$ | $CH_3$ | trans | Hydrochloride | 247-248 |
| 18-119 | 6-$CH_3$ | 4'-F | H | $CH_3$ | $CH_3$ | cis | Base | 60-62 |
| 18-200-c | 6-$CH_3$ | 4'-F | H | $C_2H_5$ | $C_2H_5$ | trans | Hydrochloride | 209-210 |
| 18-201-c | 6-$CH_3$ | 4'-F | H | $C_2H_5$ | $C_2H_5$ | cis | Hydrochloride, hydrate | 204-206 |
| 18-047-c | 6-$CF_3$ | 4'-F | H | $CH_3$ | $CH_3$ | trans | Hydrochloride | 244-245 |
| 18-150-c | 6-$CF_3$ | 4'-F | H | $CH_3$ | $CH_3$ | cis | Hydrochloride | 234-236 |
| 19-016-c | 6-$CH_3S$ | 4'-F | H | $CH_3$ | $CH_3$ | trans | Hydrochloride | 232-234 |
| 19-017-c | 6-$CH_3S$ | 4'-F | H | $CH_3$ | $CH_3$ | cis | Hydrochloride | 236-238 |
| 19-021 | 6-F | 4'-$NH_2$ | H | $CH_3$ | $CH_3$ | trans | Base | 150-152 |
| 19-022-c | 6-F | 4'-$NH_2$ | H | $CH_3$ | $CH_3$ | cis | Hydrochloride | 267-271 |
| 19-070-c | 6-F | 4'-CN | H | $CH_3$ | $CH_3$ | trans | Hydrochloride, hydrate | 199-201 |
| 19-122-c | H | 3'-Cl | 4'-Cl | H | $C_3H_7$ | trans | Hydrochloride | 214-216 |
| 19-123 | H | 3'-Cl | 4'-Cl | H | $C_3H_7$ | cis | Hydrochloride | 268-270 |

Table 2 (cont'd)

| Code No. | $R^1$ | $R^2$ | $R^3$ | $R^4$ | $R^5$ | Isomerism x) | Salt/Base | MP °C |
|---|---|---|---|---|---|---|---|---|
| 19-136-c | 6-F | 3-Cl | 4-Cl | H | $CH_3$ | Trans | Hydrochloride | 222-224 |
| 19-137-c | 6-F | 3-Cl | 4-Cl | H | $CH_3$ | Cis | Hydrochloride | 267-269 |
| 19-143-c | 6-F | 3-Cl | 4-Cl | $CH_3$ | $CH_3$ | Trans | Hydrochloride | 173-175 |
| 19-144-c | 6-F | 3-Cl | 4-Cl | $CH_3$ | $CH_3$ | Cis | Hydrochloride | 218-220 |
| 20-015-c | 6-$OCH_3$ | 3-Cl | 4-Cl | H | $CH_3$ | Trans | Hemioxalate | 231-233 |
| 20-016-c | 6-$OCH_3$ | 3-Cl | 4-Cl | H | $CH_3$ | Cis | Hydrochloride | 262-263 |
| 20-049-o | 6-$OCH_3$ | 3-Cl | 4-Cl | $CH_3$ | $CH_3$ | Trans | Oxalate | 153-157 |
| 20-050 | 6-$OCH_3$ | 3-Cl | 4-Cl | $CH_3$ | $CH_3$ | Cis | Base | 75- 77 |
| 20-030 | 6-OH | 3-Cl | 4-Cl | H | $CH_3$ | Cis | Base | 211-213 |
| 20-031-b | 6-OH | 3-Cl | 4-Cl | $CH_3$ | $CH_3$ | Trans | Hydrochloride | 217-221 |
| 20-042-c | H | 3-Cl | 4-Cl | H | $CH_3$ | (+)Trans | Hydrochloride | 184-185 |
| 20-043-c | H | 3-Cl | 4-Cl | H | $CH_3$ | (-)Trans | Hydrochloride | 182-183 |
| 19-025 | H | H | H | $CH_3$ | $CH_3$ | Cis | Hydrochloride | 196-197 |
| 19-026 | H | H | H | $CH_3$ | $CH_3$ | Trans | Hydrochloride | 186-189 |
| 19-094 | H | H | H | H | $CH_3$ | Cis | Hydrochloride | 235-237 |

In recent years it has been claimed that depressions could be related to changes in different neurone systems.

Therefore, indanamines of Formula I were tested in standard reliable tests, namely in vitro, DA-uptake inhibition, 5 HT-uptake inhibition and NA-uptake inhibitions. As in vivo tests were used antagonism against tetrabenazine induced ptosis and potentiation of 5-HTP.

All the tests show that the indanamines of Formula I have potentiating effects on both dopamine, noradrenaline and 5 HT-neurones. In contrast, no effect is shown on the cholinergic neurone system.

In more unspecific tests for antidepressant effect, like potentiation of apomorphine and antagonism against electro-shock induced convulsions and antagonism against catalepsy induced by neuroleptics, the indanamines of Formula I do also show activity.

The activity in the above outlined pharmacological tests indicates that the indanamines of Formula I would be valuable in the treatment of all kinds of depressions in mammals, including human beings, and are at the same time, due to the lack of anticholinergic effects, without the serious side effects inherent in known antidepressants.

The tests may be described as follows:

Tetrabenazine ptosis

Macrolon cages type II
Mice, male, 18-25 g

Dosage and procedure

The test substance is given i.p. in the doses 0, 1/4, 1/16 and 1/64 of the determined "i.v. $LD_{50}$". For insoluble substances the doses 0, 1/8, 1/32 and 1/128 of "i.p. $LD_{50}$" are used. 3 mice are used for each dose level. Thirty minutes after administration of test substance, tetrabenazine, 40 mg/kg, is injected i.p. Ptosis is evaluated 30 minutes after administration of tetrabenazine, the following rating scale being used: 0 = normal eyes, 1 = 1/4 closed eyes, 2 = 1/2 closed eyes, 3 = 3/4 closed eyes and 4 = completely closed eyes. Ptosis is evaluated 30 seconds after tilting the cage 3 times. The tilting is carried out in such a way that the animals slide along the floor of the cage.

The results are recorded as % inhibition of ptotic score calculated on the basis of the score of the control group. If ptotic score for the control group is $< 10$ the test is rejected and another is performed. The registration can also be done as an on line procedure.

5-HT potentiation

Macrolon cages type II
Mice, male, 18-25 g

Dosage and procedure

The test substance is given i.p. or p.o. in the doses 10, 10, 2.5, 0.63 and 0 mg/kg. Three mice are used for each dose level. One of the groups on the highest dose is used as control of the compounds own effect. If this group shows some of the symptoms mentioned below the test has to be repeated with lower doses of the test compound. Thirty minutes after administration of test substance, 5-HTP, 100 mg/kg i.v. is given (injection time 5-10 sec.). After this 5-HTP dose the animals remain unaffected. If the animals have been pretreated with a substance, which inhibits the re-uptake of 5-HT, a 5-HTP syndrome will occur. This is characterized by the following symptoms: 1) excitation, 2) tremor, and 3) abduction of the hind limbs.
After the administration of 5-HTP the animals are observed for 15 minutes and each animal is given one point for each symptom present. Thus, the maximal score for a group consisting of 3 mice is 9.

The results are stated as fractions: 0/9, 1/9, 2/9 ..... 8/9, 9/9, where 0, 1, 2 ..... 8, 9 are the number of points per group after the dose in question.

Inhibition of $^{3}H$-5-HT uptake in synaptosomes in vitro.

Rats, 180-220 g
0.32 M sucrose containing 1 mM Nialamide
Krebs-Ringer-Phosphate buffer, pH 7.4
(122 mM NaCl, 4.82 mM KCl, 972 $\mu$M $CaCl_2$, 1.21 mM $MgSO_4$, 12.7 mM $Na_2HPO_4$, 2.97 mM $NaH_2PO_4$, 162 $\mu$M EDTA-$Na_2$, 10.1 mM glucose, 1.14 mM ascorbic acid, oxygenated with pure oxygen 10 minutes before use)
Millipore filters (HAWPO 2500, 0.45 $\mu$)
5-hydroxy [G-$^{3}H$] tryptamine creatinine sulphate = $^{3}H$-5-HT, spc. act. app. 10 Ci/mmol, The Radiochemical Centre, Amersham.
Thomas Tissue Grinder, clearance 0.004-0.006 inch.

Procedure

Rats are killed by a blow on the head and exsanguinated. The brain is quickly removed, rinsed for blood and placed on a cold plate. The cerebellum, pons and medulla oblongata are removed. Rest of the brain is gently homogenized (glass teflon homogenizer) in 40 volumes of ice cold 0.32 M of sucrose containing 1 mM of nialamide. The $P_2$ fraction (synaptosomal fraction) is obtained by centrifugation (600 g, 10 min. and 25000 g, 55 min., $4^{o}C$) and suspended in 40 volumes of a modified Krebs-Ringer-Phosphate buffer, pH 7.4.

To 200 $\mu$l of the synaptosomal suspension on ice are added 3700 $\mu$l modified Krebs-Ringer-phosphate buffer, pH 7.4, containing test substances. After pre-incubation at $37^{o}C$ for 5 min., 100 $\mu$l of $^{3}H$-5-HT (final concentration in the samples = 10 nM) are added and the samples are incubated for 10 min. at $37^{o}C$. The incubation is terminated by filtering the samples under vacuum through Millipore filters with a wash of 5 ml buffer containing 10 $\mu$M of unlabelled 5-HT. After solubilizing the filters in 1 ml of cellosolve, 10 ml of Instagel are added, and the radioactivity is determined by liquid and scintillation counting. The unspecific binding and passive transport of $^{3}H$-5-HT is determined by incubating control samples on ice instead of at $37^{o}C$. All experiments are performed in triplicate. The mean of all control samples are calculated.

The measured cpm in the samples are plotted against drug concentration on semilogarithmic paper, and the best fitting s-shaped curve drawn. The IC50-values are determined as the concentrations, at which the uptake are 50 per cent of the uptake in control sample -uptake in ice samples.

$^{3}H$-DA uptake in striatal synaptosomes in vitro.

Rats, 180-220 g
0.32 M sucrose containing 1 mM Nialamide
Krebs-Ringer-phosphate buffer, pH 7.4
(122 mM NaCl, 4.8 mM KCl, 972 $\mu$M $CaCl_2$, 1.2 mM $MgSO_4$, 12.7 mM $Na_2HPO_4$, 3.0 mM $NaH_2PO_4$, 162 $\mu$M EDTA-$Na_2$, 1.14 mM ascorbic acid, 10.1 mM glucose, oxygenated with pure oxygen 10 minutes before use) Millipore filters (HAWPO 2500, 0.45 $\mu$)
3,4-dihydroxyphenylethylamine [ethyl-1-$^{3}H$(N)] = $^{3}H$-DA, spc. act. app. 15 Ci/mmol, New England Nuclear
Thomas Tissue Grinder, clearance 0.004-0.006 inch.

Procedure

Rats are killed by a blow to their head, exsanguinated and their brains removed. The brain is placed on a precooled glassplate, and the two corpura striata are dissected out and gently homogenized in 40 volumes of ice cold 0.32 M sucrose containing 1 mM of nialamide using a hand homogenizer with teflon pestle. The $P_2$-fraction (synaptosomal fraction) is obtained by centrifugation (600 g, 10 min., 25000 g, 55 min. 4°C) and suspended in 40 volumes of a modified Krebs-Ringer-phosphate buffer, pH 7.4. To 200 µl of the synaptosomal fraction on ice are added 3700 µl modified Krebs-Ringer-phosphate buffer - containing test compounds. After a preincubation at 37°C for 5 min. 100 µl of $^3$H-DA (final conc. 12.5 nM) are added, and the samples are incubated for 5 min. at 37°C. The incubation is terminated by filtering the samples under vacuum through Millipore filters with a wash of 5 ml buffer containing 10 µM of unlabelled DA. After solubilizing the filters in 1 ml of cellosolve the radioactivity is determined by liquid scintillation counting after the addition of 10 ml of Insta-Gel. The unspecific binding of $^3$H-DA is determined by incubating control samples on ice instead of at 37°C.

All experiments are performed in triplicate.

The mean of all control samples and ice samples are calculated. The measured cpm in the samples are plotted against drug concentration on semilogarithmic paper, and the best fitting S-shaped curve drawn. The IC50-values are determined as the concentrations, at which the uptake are 50 per cent of the uptake in control samples - uptake in ice samples.

Inhibition of $^3$H-1-NA uptake in synaptosomes in vitro.

Rats, 180-220 g

0.32 M sucrose containing 1 mM Nialamide

Krebs-Ringer-Phosphate buffer, pH 7.4

(122 mM NaCl, 4.82 mM KCl, 972 µM $CaCl_2$, 1.21 mM $MgSO_4$, 12.7 mM $Na_2HPO_4$, 2.97 mM $NaH_2PO_4$, 162 µM EDTA-$Na_2$, 10.1 mM glucose, 1.14 mM ascorbic acid, oxygenated with pure oxygen 10 min. before use)

Millipore filters (HAWPO 2500, 0.45 µ)

Norepinephrine levo-[7,8-$^3$H(N)] =

$^3$H-1-NA, spc. act. app. 30 Ci/mmol, New England Nuclear.

Thomas Tissue Grinder, clearance 0.004-0.006 inch.

Procedure

Rats are killed by decapitation and exsanguinated. The brain is quickly removed, rinsed for blood and placed in cold saline. Occipital cortex tissue from both sites is removed, and is gently homogenized (glass teflon homogenizer) in 40 volumes of ice cold 0.32 M of sucrose containing 1 mM of nialamide. The $p_2$ fraction (synaptosomal fraction) is obtained by centrifugation (600 g, 10 min. and 25000 g, 55 min. 4°C) and suspended in 25 volumes of a modified Krebs-Ringer-phosphate buffer, pH 7.4.

To 200 $\mu$l of the synaptosomal suspension on ice are added 3700 $\mu$l modified Krebs-Ringer-phosphate buffer, pH 7.4, containing test substances. After preincubation at 37°C for 5 min. 100 $\mu$l of $^3$H-1-NA (final concentration in the samples = 10 nM) are added and the samples are incubated for 30 min at 37°C. The incubation is terminated by filtering the samples under vacuum through Millipore filters with a wash of 5 ml buffer containing 10 $\mu$M of unlabelled 1-NA. After solubilizing the filters in 1 ml of cellosolve, 10 ml of Instagel, lumagel or picofluor 15 are added, and the radioactitivy is determined by liquid scintillation counting. The unspecific binding and passive transport of $^3$H-1-NA is determined by incubating control samples with 10 $\mu$M of Lu 5-003-c.
All experiments are performed in triplicate.
The mean of all control and Lu 5-003 samples are calculated.

The cpm measured in the samples are plotted against drug concentration on semilogarithmic paper, and the best fitting s-shaped curve drawn. The IC50-values are determined as the concentrations, at which the uptake are 50 per cent of the uptake in control samples - uptake in Lu 5-003 samples.

In Table 3 the compounds of the invention have been compared with Lu 19-025-c and Lu 19-026-c, which are respectively the cis- and trans-isomers of the compound of Formula I wherein $R^1$, $R^2$ and $R^3$ are hydrogen, $R^4$ and $R^5$ are methyl and $R^6$ is hydrogen, and moreover compared with the known antidepressants amitriptyline and nomiphensine.

The result of the test will appear from the following TABLE 3:

TABLE 3

0076669

| Lu-Code No. | Tetrabenazine ptosis, $ED_{50}$ /u mol/kg i.p. | 5-HTP potentation, $ED_{50}$ /u mol/kg i.p. | Uptake Inhibition $IC_{50}$ (nM) DA | 5-HT | NA |
|---|---|---|---|---|---|
| 13-114-c | 28 | >130 | 620 | 18 | 350 |
| 13-164-o | 22 | 57 | | | |
| 18-047-c | 166 | | 240 | 43 | |
| 18-118 | 83 | 5 | 28 | 0.64 | 24 |
| 18-119 | 45 | >37 | 71 | 1.4 | 20 |
| 18-122-c | 14 | 10 | 27 | 0.37 | 2.4 |
| 18-125-c | 41 | 44 | 100 | 9.8 | 73 |
| 18-136-c | 23 | 19 | 2000 | 0.58 | 90 |
| 18-137-c | 23 | 19 | 860 | 0.03 | 34 |
| 18-143-c | >109 | | 300 | 5400 | 860 |
| 18-144-c | >109 | | 840 | 6700 | |
| 18-147 | > 147 | 21 | 440 | 1.2 | 90 |
| 18-148-c | >118 | | 1600 | 440 | |
| 18-149-c | 52 | | 68 | 140 | 120 |
| 18-150-c | >111 | 101 | 340 | 4.3 | 350 |
| 18-187-c | 52 | 19 | 2.1 | 2.8 | 0.56 |
| 18-188-c | >108 | | 120 | 13 | |
| 18-200-c | 63 | | 210 | | |
| 17-201-c | >114 | | 470 | | |
| 19-005-c | 3.9 | 6.7 | 0.59 | 0.46 | 0.27 |
| 19-006-c | 5.8 | 4.7 | 5.2 | 0.44 | 20 |
| 19-007-c | 7.8 | 45 | 320 | | |
| 19-008-c | >137 | 25 | 250 | | |
| 19-016-c | 35 | 39 | 170 | 4.5 | 61 |
| 19-017-c | 27 | 23 | 150 | <1 | 16 |
| 19-021 | 9 | 15 | 190 | 330 | 75 |
| 19-022-c | 19 | 14 | 170 | | |
| 19-025-c | >143 | >36 | 340 | 9.5 | 41 |
| 19-026-c | 17 | >35 | 600 | 150 | 130 |
| 19-030-o | 16 | >27 | 7.4 | 15 | 1.4 |
| 19-034 | 9 | 11 | 130 | 1.7 | 130 |
| 19-040-c | > 7 | 26 | 1.5 | 2.3 | 0.32 |
| 19-041-c | > 117 | 24 | 62 | | |

TABLE 3 cont'd



| Lu-Code No. | Tetrabenazine ptosis, $ED_{50}$ /u mol/kg i.p. | 5-HTP potentation, $ED_{50}$ /u mol/kg i.p. | Uptake Inhibition $IC_{50}$ (nM) DA | 5-HT | NA |
|---|---|---|---|---|---|
| 19-042-c | >25 | >100 | 150 | 720 | 73 |
| 19-043-c | >100 | >100 | 180 | | |
| 19-056 | 10 | 8 | 54 | 0.04 | 28 |
| 19-057 | 3.9 | 12 | 83 | 0.14 | 4.3 |
| 19-070-c | 45 | 25 | | | |
| 19-071-c | >25 | >25 | | | |
| 19-072-c | >99 | >99 | | | |
| 19-073-c | >111 | >111 | | | |
| 19-074-c | 75 | 270 | | | |
| 19-078-c | 33 | 142 | | | |
| 19-079-o | >85 | >85 | | | |
| 19-082-c | 104 | >99 | | | |
| 19-083-o | >83 | >83 | | | |
| 19-085-c | 51 | 47 | | | |
| 19-086-c | 32 | 30 | | | |
| 19-091-c | >137 | 55 | | | |
| 19-092-c | 34 | 63 | | | |
| 19-094 | >154 | 211 | 1400 | 210 | 340 |
| 19-095-c | 7.1 | 73 | | | |
| 19-096-c | 13 | 42 | | | |
| 19-097-c | | 94 | | | |
| 19-098-c | | 22 | | | |
| 19-099-c | 19 | 67 | 510 | 0.91 | 15 |
| 19-100-c | 4 | 37 | | | |
| 19-101-c | 24 | 51 | | | |
| 19-102-c | 4 | 8.8 | 2.5 | 0.28 | 0.30 |
| 19-103-c | 29 | 6.6 | 67 | 0.16 | 8.8 |
| 19-104-c | 23 | 63 | | | |
| 19-105-o | 12 | 95 | | | |
| 19-108-c | 79 | 72 | | | |
| 19-109-c | 6.3 | >36 | | | |
| 19-110-c | 142 | 124 | | | |
| 19-111-c | 45 | 92 | | | |
| 19-113-c | 98 | 71 | | | |
| 19-114 | 14 | 26 | | | |
| 19-115-c | 10 | 43 | | | |

TABLE 3

| Lu-Code No. | Tetrabenazine ptosis, $ED_{50}$ /u mol/kg i.p. | 5-HTP potentation, $ED_{50}$ /u mol/kg i.p. | Uptake Inhibition $IC_{50}$ (nM) DA | 5-HT | NA |
|---|---|---|---|---|---|
| 19-116-c | 85 | 78 | | | |
| 19-119 | >155 | 34 | 5.6 | 4.0 | 0.51 |
| 19-120-c | >34 | 23 | 120 | 0.65 | 24 |
| 19-121 | 66 | 18 | 140 | 0.64 | 23 |
| 19-122-c | 68 | 87 | | | |
| 19-123-c | >112 | >112 | | | |
| 19-124 | 43 | 109 | 190 | 0.34 | 4.4 |
| 19-136-c | 33 | 27 | | | |
| 19-137-c | 15 | 34 | | | |
| 19-143-c | >111 | 14 | | | |
| 19-144-c | >111 | 29 | | | |
| 20-015-o | 7 | 14 | 2.5 | 0.62 | 0.67 |
| 20-016-o | 14 | 3 | 19 | 0.53 | 19 |
| 20-030 | 15 | 59 | 4.1 | 0.11 | 6.3 |
| 20-031-b | 97 | 18 | | | |
| 20-042-c | 7.6 | 11 | 1.9 | 0.96 | 0.56 |
| 20-043-c | 3.8 | 16 | 2.2 | 0.37 | 0.58 |
| 20-049-o | 5.9 | 37 | 10 | | 16 |
| 20-050 | 119 | 26 | 15 | | 3.0 |
| Citalo-pram | 20 | 3.3 | 41000 | 1.8 | 8.800 |
| Nomi-phensine | 4.3 | >56 | 48 | 830 | 6.6 |
| Amitrip-tyline | 7 | | 5400 | 40 | 24 |

The compounds of Formula I and the non-toxic acid addition salts thereof may be administered to animals such as dogs, cats, horses, sheeps or the like, including human beings, both orally and parenterally, and may be used for example in the form of tablets, capsules, powders, syrups or in the form of the usual sterile solutions for injection. - Results upon administration to human beings have been very gratifying.

Most conveniently the compounds of Formula I are administered orally in unit dosage form such as tablets or capsules, each dosage unit containing the free amine or a non-toxic acid addition salt of one of the said compounds in a amount of from about o.10 to about 100 mg, most preferably, however, from about 5 to 50 mg, calculated as the free amine, the total daily dosage usually ranging from about 1.o to about 500 mg. The exact individual dosages as well as daily dosages in a particular case will, of course, be determined according to established medical principles under the direction of a physician.

When preparing tablets, the active ingredient is for the most part mixed with ordinary tablet adjuvants such as corn starch, potato starch, talcum, magnesium stearate, gelatine, lactose, gums, or the like.

When the compound of Formula I is an ester, preferably a decanoic acid ester, palmitic acid ester or a behenic acid ester, the composition may advantageously be an oily solution for injection, and such solutions often have a very prolonged effect when compared with the corresponding unesterified compound.

Typical examples of formulas for composition containing trans N-methyl-3(3,4-dichlorophenyl)-1-indanamine (called Lu 19-005-c for short) as the active ingredient, are as follows:

1) Tablets containing 5 milligrams of Lu 19-005-c calculated as the free base:

| | |
|---|---|
| Lu 19-005-c | 5 mg |
| Lactose | 18 mg |
| Potato starch | 27 mg |
| Saccharose | 58 mg |
| Sorbitol | 3 mg |
| Talcum | 5 mg |
| Gelatine | 2 mg |
| Povidone | 1 mg |
| Magnesium stearate | 0.5 mg |

2) Tablets containing 50 milligrams of Lu 19-005-c calculated as the free base:

| | |
|---|---|
| Lu 19-005-c | 50 mg |
| Lactose | 16 mg |
| Potato starch | 45 mg |
| Saccharose | 106 mg |
| Sorbitol | 6 mg |
| Talcum | 9 mg |
| Gelatine | 4 mg |
| Povidone | 3 mg |
| Magnesium stearate | 0.6 mg |

3) Syrup containing per milliliter:

| | |
|---|---|
| Lu 19-19005-c | 10 mg |
| Sorbitol | 500 mg |
| Tragacanth | 7 mg |
| Glycerol | 50 mg |
| Methyl-paraben | 1 mg |
| Propyl-paraben | 0.1 mg |
| Ethanol | 0.005 ml |
| Water | ad 1 ml |

4) Solution for injection containing per milliliter:

| | |
|---|---|
| Lu 19-005-c | 50 mg |
| Acetic acid | 17.9 mg |
| Sterile water | ad 1 ml |

5) Solution for injection containing per milliliter:

| | |
|---|---|
| Lu 19-005-c | 10 mg |
| Sorbitol | 42.9 mg |
| Acetic acid | 0.63 mg |
| Sodium hydroxide | 22 mg |
| Sterile water | ad 1 ml |

Any other pharmaceutical tableting adjuvants may be used provided that they are compatible with the active ingredient, and additional compositions and dosage forms may be similar to those presently used for thymoleptics, such as amitriptyline, nortriptyline or imipramine.

Also combinations of the compounds of Formula I as well as their non-toxic acid salts with other active ingredients, especially other neuroleptics, thymoleptics, tranquilizers, analgetics or the like, fall within the scope of the present invention.

As previously stated, when isolating the compounds of Formula I in the form of an acid addition salt the acid is preferably selected so as to contain an anion which is non-toxic and pharmacologically acceptable, at least in usual therapeutic doses. Representative salts which are included in this preferred group are the hydrochlorides, hydrobromides, sulphates, acetates , phosphates, nitrates, methanesulphonates, ethane-sulphonates, lactates, citrates, tartrates or bitartrates, pamoates and maleates of the amines of Formula I. Other acids are likewise suitable and may be employed if desired. For example: fumaric, benzoic, ascorbic, succinic, salicylic, bismethylenesalicylic, propionic, gluconic, malic, malonic, mandelic, cannamic, citraconic, stearic, palmitic, itaconic, glycolic, benzenesulphonic, and sulphamic acids may also be employed as acid addition saltforming acids.

When it is desired to isolate a compound of the invention in the form of the free base, this may be done according to conventional procedure as by dissolving the isolated or unisolated salt in water, treating with a suitable alkaline material,

extracting the liberated free base with a suitable organic solvent drying the extract and evaporating to dryness or fractionally distilling to effect isolation of the free basic amine.

The invention also comprises a method for the alleviation, palliation, mitigation or inhibition of the manifestations of certain physiological-psychological abnormalies of animals, including depressions, by administering to a living animal body, including human beings, an adequate quantity of a compound of Formula I or a non-toxic acid addition salt thereof. An adequate quantity would be from about o.ool mg to about 10 mg per kg of body weight in each unit dosage, and from about o.oo3 milligrams to about 7 milligrams /kg of body weight per day.

It is to be understood that the invention is not limited to the exact details of operation or exact compound or compositions shown and described, as obvious modifications and equivalents will be apparent to one skilled in the art.

I CLAIM:

-1-

3-Phenyl-1-indanamines having the following formula:

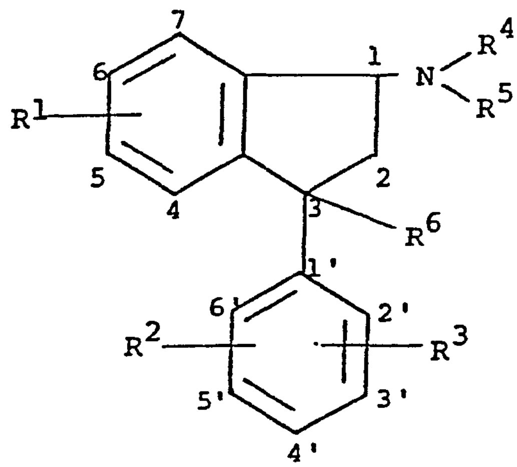

I

wherein $R^1$, $R^2$ and $R^3$ each represents hydrogen, halogen, lower alkyl, lower alkyloxy, hydroxy, lower alkylmercapto, cyano, trifluoromethyl, amino, acylamino, lower alkylamino or nitro, provided that $R^1$, $R^2$ and $R^3$ may not represent hydrogen at the same time, $R^4$ and $R^5$ represent each hydrogen, lower alkyl or lower alkenyl, optionally substituted with hydroxy, optionally substituted cycloalkyl (3-6 C-atoms) or optionally substituted phenyl, $R^6$ represents hydrogen or hydroxy, any hydroxy group present in the indanamine of Formula I being optionally esterified with an aliphatic carboxylic acid having from two to twenty-four carbon atoms inclusive, as well as their pharmaceutically acceptable acid addition salts.

-2-A compound according to Claim 1, which is a trans-isomer of a compound wherein $R^1$ is hydrogen or methoxy, $R^2$ and $R^3$ each are chlorine in the 3-and 4-position respectively, $R^4$ is methyl or ethyl, $R^5$ is hydrogen or ethyl and $R^6$ is hydrogen.

-3-

A compound according to Claim 1, wherein $R^1$ is hydrogen, $R^2$ is 2-chloro or hydrogen, $R^3$ is chloro or bromo, $R^4$ is hydrogen or methyl, $R^5$ is methyl and $R^6$ is hydrogen.

-4-

N-methyl-3-(3,4-dichlorophenyl)-1-indanamine, its isomers and pharmaceutically acceptable acid addition salts thereof.

-5-N,N-dimethyl-3-(3,4-dichlorophenyl)-1-indanamine, its isomers and pharmaceutically acceptable acid addition salts thereof.

-6-N,N-dimethyl-3-(2,4-dichlorophenyl)-1-indanamine, its isomers and pharmaceutically acceptable acid addition salts thereof.

-7-N,N-dimethyl-3-(4'-bromophenyl)-1-indanamine, its isomers and pharmaceutically acceptable acid addition salts thereof.

-8-A pharmaceutical composition comprising a therapeutically effective amount of a compound of Claim 1 together with a pharmaceutical carrier or excipient.

-9-A pharmaceutical composition according to Claim 8, wherein the active ingredient is present in an amount of from 0.1 - 100 milligrams per unit dose.

-10-A method for the preparation of the novel indanamines of Formula I as defined in Claim 1, which comprises:

a) reacting a compound of the following formula:

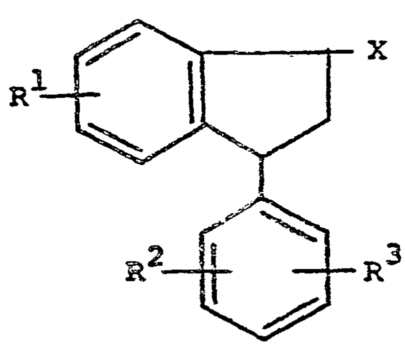

II

wherein $R^1$, $R^2$ and $R^3$ are as defined above, and X is halogen, preferably chlorine, or $-OS_2R$, wherein R is $CH_3$ or p-tolyl, with an amine of formula $HNR^4R^5$, wherein $R^4$ and $R^5$ are as defined above,

or

b) reducing a Schiff-base of the following formula:

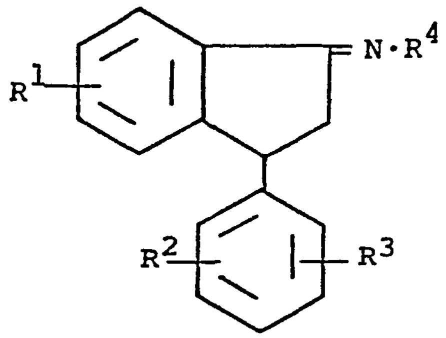

III

with a reducing agent such as $NaBH_4$ or hydrogen in the presence of a metal catalyst, such as palladium on charcoal or $PtO_2$ ,

or

c) alkylating a compound of the following formula:

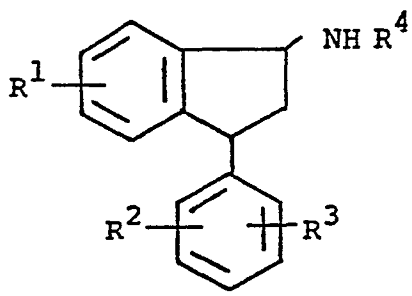

IV

wherein $R^1$, $R^2$, $R^3$ and $R^4$ are as defined above, with either a compound $R^5 \cdot X$ wherein $R^5$ and X are as defined above, or by reductive alkylation with an aldehyde $R^{5'} \cdot CHO$ or ketone $R^{6'}CO \cdot R^{7'}$ or a carboxylic acid $-NaBH_4$ complex, wherein $R^{5'}$ represents optionally substituted alkyl or alkenyl having one carbon atom less than $R^5$, and $R^6$ and $R^7$ together with the carbon atom from the keto group form a branched optionally substituted lower alkyl-or alkenyl group,

or

d) reducing a compound of the following formula:

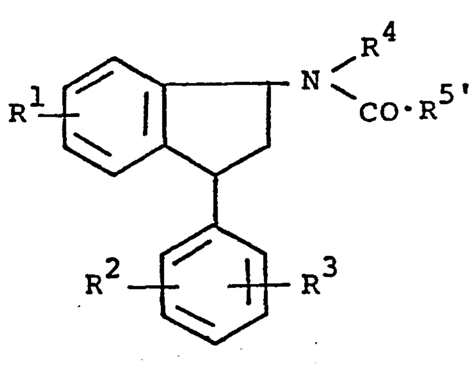

V

wherein $R^1$, $R^2$, $R^3$, $R^4$ and $R^5$ are as defined above, or with a reducing agent such as $LiAlH_4$,

or

e) reacting an indanone of the following formula:

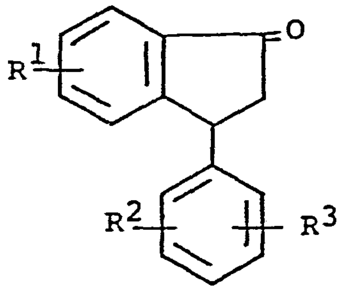

VI

wherein $R^1$, $R^2$ and $R^3$ are as defined above, with an amine of the formula $R^4 \cdot NH R^5$, wherein $R^4$ and $R^5$ are as defined above, in the presence of hydrogen and a metal catalyst,

or

f) reacting a compound of the following formula:

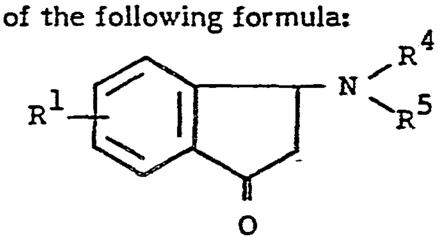

VII

wherein $R^1$, $R^4$ and $R^5$ are as defined above, with a Grignard- or lithium reagent of the formula 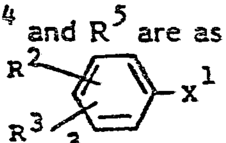
wherein $R^2$ and $R^3$ are as defined above, and $X^1$ is Mghal or Li, followed by hydrolysis of the complex formed,

or

g) reducing a compound of the following formula:

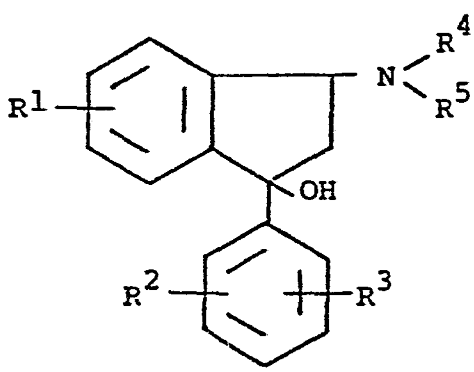

VIII

wherein $R^1$, $R^2$, $R^3$, $R^4$ and $R^5$ are as defined above,

or

h) reducing an indene of the following formula:

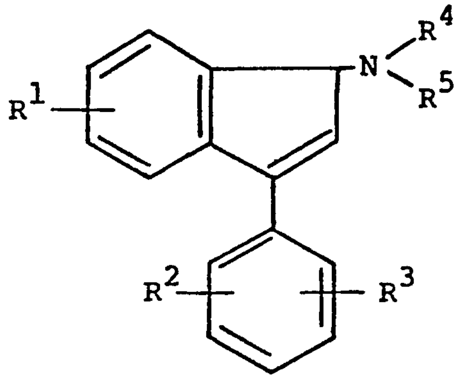

IX

wherein $R^1$, $R^2$, $R^3$, $R^4$ and $R^5$ are as defined above,

or

i) cleaving a methoxy group in a compound of Formula I, wherein one of $R^1$, $R^2$ or $R^3$ is methoxy, to give the corresponding hydroxy substituted compound,

whereupon the indanamine of Formula I formed by the reaction is isolated in the form of the free base or in the form of a pharmaceutically acceptable acid addition salt and, if desired, separated in the individual isomers in conventional manner, any hydroxy group present optionally being esterified with a reactive derivative of an aliphatic carboxylic acid having from one to twenty-four carbon atoms inclusive.

European Patent Office

# EUROPEAN SEARCH REPORT

0076669
Application number

EP 82 30 5244

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | DE-C- 955 594 (SCHERING) *The whole document* | 1,8,9, 10 | C 07 C 87/457<br>C 07 C 87/60<br>C 07 C 91/16<br>C 07 C 91/28<br>C 07 C 93/14<br>C 07 C 121/78<br>C 07 C 149/42<br>A 61 K 31/135 |
| X | GB-A- 971 917 (SCHERING) *Claims; page 1, page 3* | 1,8,9 | |
| D,X | DE-A-2 339 715 (HOECHST) *Claims* | 1,8,9 | |
| X | EP-A-0 035 363 (KEFALAS) *Claims* | 1,8,9, 10 | |
| | ----- | | TECHNICAL FIELDS SEARCHED (Int. Cl. 3) |
| | | | C 07 C 87/00<br>C 07 C 91/00<br>C 07 C 93/00<br>C 07 C 121/00<br>C 07 C 149/00<br>A 61 K 31/00 |

The present search report has been drawn up for all claims

| Place of search | Date of completion of the search | Examiner |
|---|---|---|
| THE HAGUE | 05-11-1983 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document